# EUROPEAN PATENT APPLICATION

(11) **EP 4 180 054 A1**
(43) Date of publication of application: **17.05.2023**
(21) Application number: 21842083.4
(22) Date of filing: 12.07.2021
(51) Int. Cl.: A61K 38/04, A61K 31/395, A61P 43/00, C12N 5/0789, A61M 1/02, A61M 1/34

(54) **COMBINATION THERAPY OF SUBSTANCE-P FOR MOBILIZATION OF HEMATOPOIETIC STEM CELLS**

(30) Priority: 13.07.2020 KR 20200086108
(71) Applicant: Elphis Cell Therapeutics, Seoul 02455 (KR)
(72) Inventor: SON, Young Sook, Seoul 06305 (KR); HONG, Hyun Sook, Seoul 05619 (KR); PARK, Jeong Seop, Seoul 02460 (KR)
(74) Representative: Schön, Christoph
(86) International application number: PCT/KR2021/008882
(87) International publication number: WO 2022/014985

(57) **Abstract**

The present invention relates to: a combination therapy and a combination product that are for the mobilization of a large amount of hematopoietic stem cells in blood and include substance-P and AMD3100 or a pharmaceutically acceptable salt thereof; a method for collecting a large amount of mobilized hematopoietic stem cells, the method comprising a step for isolating a blood fraction containing the mobilized hematopoietic stem cells from peripheral blood obtained from an individual sequentially administered with substance P and AMD3100 or a pharmaceutically acceptable salt thereof; and a use of a combined product containing substance P and AMD3100 or a pharmaceutically acceptable salt thereof for the mobilization of a large amount of hematopoietic stem cells in blood.

## Description

### [Technical Field]

The present invention provides a combination therapy and combination product for mobilization of a large amount of hematopoietic stem cells in blood, comprising substance P and AMD3100 or a pharmaceutically acceptable salt thereof; a method for collecting a large amount of mobilized hematopoietic stem cells, comprising separating blood fractions containing mobilized hematopoietic stem cells from peripheral blood obtained from a subject to which substance P and AMD3100 or a pharmaceutically acceptable salt thereof were sequentially administered; and use of a combination comprising substance P and AMD3100 or a pharmaceutically acceptable salt thereof for mobilization of a large amount of hematopoietic stem cells in blood.

### [Background Art]

Transplantation of hematopoietic stem cells (HSCs) mobilized or migrated from bone marrow to peripheral blood into patients suffering from a blood disease has replaced traditional bone marrow transplantation. Some clinical implementations for mobilization of hematopoietic stem cells from bone marrow to peripheral blood have been performed with a dosing regimen of at least 5 consecutive days of recombinant granulocyte colony stimulating factor (G-CSF) thought to stimulate the production of proteases that cleave the CXCR4/SDF-1 interaction. Granulocyte colony stimulating factor (G-CSF) is a glycoprotein that stimulates the survival, proliferation, differentiation and function of neutrophil granulocyte progenitor cells and mature neutrophils.

However, G-CSF has been reported to be ineffective in large patient cohorts and associated with several side effects (e.g., bone pain, spleen enlargement and, rarely, spleen rupture, myocardial infarction and cerebrovascular diseases). In addition, G-CSF has been reported to have a risk of causing thrombotic complications when administered for a long time since G-CSF has been confirmed to induce a strong inflammatory response and induces mobilization of various types of blood cells, in addition to stem cells, into the peripheral blood. In particular, since G-CSF needs subcutaneous injection for at least 5 consecutive days, and the patient's thigh pain is accompanied during such a process, caution is needed in clinical use of G-CSF. In addition, the main mechanism of G-CSF is the stimulation of neutrophil production, and the treatment of neutrophenia is a major target thereof.

The intrinsic disadvantages of G-CSF have led to efforts to identify alternative mobilization methods based on small molecules. For example, the FDA-approved CXCR4 antagonist AMD3100 (Plerixafor; Mozobil^{™}) is known to have limited toxicity issues and rapidly mobilize hematopoietic stem cells. However, clinical mobilization using AMD3100 was found to be effective only when combined with G-CSF. The current clinical regimen/dose of G-CSF and ADM3100 combination therapy is to inject G-CSF once a day for 5 consecutive days by subcutaneous injection, and intraperitoneally inject AMD3100 approximately 4 to 6 hours after the last injection. Blood is collected 1 hour after the administration of AMD3100, and the migration of hematopoietic stem cells into peripheral blood at this time is known to be at its maximum.

However, the response to G-CSF and ADM3100 combination therapy varies between patients, and it is known that there are non-responders who have no change in the level of hematopoietic stem cells in blood. Also, even if G-CSF is clinically the most widely used migrator, G-CSF has additional disadvantages (e.g., potential toxic side effects, induction of a strong inflammatory response, relatively long course of treatment (e.g., injection for 5 to 7 consecutive days) and variable response among patients).

Therefore, the search for a rapid, selective and G-CSF-independent mobilization mode remains a subject of clinical interest. The research and development of migrators replacing G-CSF are currently in progress.

Shun He *et al.* reported that the combination administration of FLT3 ligand (FLT3L) and AMD3100 in murine increases mobilization of hematopoietic stem cells *(see* Shun He et al., "FLT3L and Plerixafor Combination Increases Hematopoietic Stem Cell Mobilization and Leads to Improved Transplantation Outcome," Biol Blood Marrow Transplant (2014), 20(3): 309-313).

In addition, MP Rettig *et al.* discloses a method for mobilizing hematopoietic stem cells (HSCs) and hematopoietic progenitor cells (HSPCs) by the combination administration of an integrin α₄β₁ (very late antigen 4 (VLA-4)) inhibitor and a CXCR4 inhibitor. In particular, it has been reported that the combination administration of a VLA-4 antagonist BIO5192 and AMD3100 in murine has an additive effect on mobilization of hematopoietic progenitor cells (HPSC) *(see* MP Rettig et al., "Mobilization of hematopoietic stem and progenitor cells using inhibitors of CXCR4 and VLA-4," Leukemia (2012), 26: 34-53).

In addition, Kasia Mierzejewska *et al.* reported that the combination administration of phenylhydrazine (PHZ) and the CXCR4 inhibitor AMD3100 in mice increased mobilization of hematopoietic stem cells *(see* Kasia Mierzejewska et al., "Sphingosine-1-phosphate-Mediated Mobilization of Hematopoietic Stem/Progenitor Cells during Intravascular Hemolysis Requires Attenuation of SDF-1-CXCR4 Retention Signaling in Bone Marrow," Hindawi Publishing Corporation, BioMed Research International (2013), Article ID 814549, p. 5).

In addition, Korean Laid-open Patent Publication No. 2017-0105514 discloses a method for leaving and releasing HSCs using an α₉ (alpha 9) integrin antagonist and a CXCR4 antagonist.

However, despite the research and development on the migrators that replace G-CSF, the combination administration of G-CSF and AMD3100 is currently recognized as the only standard treatment method.

Substance P is a neuropeptide comprising eleven (11) amino acids expressed in sensory neurons, macrophages, eosinophils, endothelial cells, corneal cells such as epithelial cells and keratocytes, and granulation tissue. Several reports have suggested that substance P is involved in neuro-immune communication for hematopoietic regulation. The nerve fibers of substance P nerves are distributed in the bone marrow stroma, and substance P transmits a signal through the surface receptor NK-1 of bone marrow stromal cells to stimulate the bone marrow stromal cells such that the bone marrow stromal cells as suppliers produce stem cell factors and interleukin-1 which are beneficial for promoting hematopoiesis.

With regard to the role of substance P in migration of mesenchymal stem cells and repopulation of mesenchymal stem cells in the above-described study, the inventors of the present invention identified, in Korean Patent No. 10-593397, uses of substance P as a wound healing agent or a wound healing promoter, a mesenchymal stem cell releasing agent or a releasing promoter, a mesenchymal stem cell proliferating agent or a proliferation promoter, and a wound healing agent or a wound healing promoter therefrom.

However, nothing has been known about the combination therapy of substance P and AMD3100 until now.

### [Summary of Invention]

### [Technical Problem]

Since G-CSF requires a relatively long treatment process (e.g., injection for 5 to 7 consecutive days) and has side effects that induce a strong inflammatory response, the present inventors intend to provide a combination therapy that can replace a conventional G-CSF and ADM3100 combination therapy for mobilization of hematopoietic stem cells in blood.

### [Solution to Problem]

The inventors of the present invention have studied to solve the above-described problem and, as a result, have completed the present invention under the discovery that, when substance P instead of G-CSF is used in combination with AMD3100, substance P produces the same effect of mobilizing hematopoietic stem cells to peripheral blood with only one intravenous injection thereof, and substance P does not have the function of a strong neutrophil mobilizer possessed by G-CSF such that the side effects of the inflammatory reaction are much less, and substance P may mobilize a larger amount of hematopoietic stem cells into blood, as compared to a conventional G-CSF and ADM3100 combination therapy.

Accordingly, an object of the present invention is to provide a combination therapy for mobilization of a large amount of hematopoietic stem cells in blood, comprising substance P and AMD3100 or a pharmaceutically acceptable salt thereof.

Another object of the present invention is to provide a combination product for mobilization of a large amount of hematopoietic stem cells in blood, comprising substance P and AMD3100 or a pharmaceutically acceptable salt thereof.

Another object of the present invention is to provide a method for collecting a large amount of mobilized hematopoietic stem cells, comprising separating blood fractions containing mobilized hematopoietic stem cells from peripheral blood obtained from a subject to which substance P and AMD3100 or a pharmaceutically acceptable salt thereof were sequentially administered.

Another object of the present invention is to provide use of a combination comprising substance P and AMD3100 or a pharmaceutically acceptable salt thereof for mobilization of a large amount of hematopoietic stem cells in blood.

### [Effects of Invention]

The combination therapy comprising substance P and AMD3100 or a pharmaceutically acceptable salt thereof of the present invention may mobilize hematopoietic stem cells to peripheral blood with only one intravenous injection of substance P to significantly reduce the patient's burden during treatment, as compared to the conventional G-CSF and ADM3100 combination therapy which requires a long treatment process (e.g., injection for 5 to 7 consecutive days). Substance P does not have the function of a strong neutrophil mobilizer possessed by G-CSF such that the side effects of the inflammatory reaction are much less, and may mobilize a larger amount of hematopoietic stem cells in blood, as compared to the conventional G-CSF and ADM3100 combination therapy. In addition, the blood fractions collected by the collection method of the present invention comprise a large amount of mobilized hematopoietic stem cells and, thus, may be transplanted or re-injected into a patient suffering from a blood disease and used to prevent or treat various diseases requiring hematopoietic stem cells.

### [Brief Description of Drawings]

FIG. 1 is a graph showing the results of intravenously injecting substance P (SP) and G-CSF, respectively, into rabbits and then comparing the levels of hematopoietic stem cells in the blood of the same animals for 3 days.
FIG. 2 is a graph showing the results of intravenously injecting substance P (SP) and G-CSF, respectively, into rabbits and then comparing the ratio of immune cells in the blood of the same animals for 3 days.
FIG. 3 is a schematic diagram showing the administration schedule of a conventional G-CSF and ADM3100 combination therapy and the administration schedule of the substance P and AMD3100 combination therapy of the present invention.
FIG. 4 is a graph showing the results of administration to mice according to the administration schedule of a conventional G-CSF and ADM3100 combination therapy and the administration schedule of the substance P and AMD3100 combination therapy of the present invention, respectively, and then comparing the levels of hematopoietic stem cells in the collected blood.

### [Embodiments for Practicing Invention]

The inventors of the present invention have found that, when substance P instead of G-CSF is used in combination with AMD3100, substance P produces the same effect of mobilizing hematopoietic stem cells to peripheral blood with only one intravenous injection thereof, and substance P does not have the function of a strong neutrophil mobilizer possessed by G-CSF such that the side effects of the inflammatory reaction are much less, and substance P may mobilize a larger amount of hematopoietic stem cells into blood, as compared to a conventional G-CSF and ADM3100 combination therapy.

Accordingly, the present invention provides a combination therapy for mobilization of a large amount of hematopoietic stem cells in blood, comprising substance P and AMD3100 or a pharmaceutically acceptable salt thereof.

The present invention also provides a combination product for mobilization of a large amount of hematopoietic stem cells in blood, comprising substance P and AMD3100 or a pharmaceutically acceptable salt thereof.

The present invention also provides a method for collecting a large amount of mobilized hematopoietic stem cells, comprising separating blood fractions containing mobilized hematopoietic stem cells from peripheral blood obtained from a subject to which substance P and AMD3100 or a pharmaceutically acceptable salt thereof were sequentially administered.

The present invention also provides use of a combination comprising substance P and AMD3100 or a pharmaceutically acceptable salt thereof for mobilization of a large amount of hematopoietic stem cells in blood.

As used herein, the term "substance P" is used in the same sense as the term "substance-P" or "SP."

As used herein, the term "hematopoietic stem cell" is used in the same sense as the term "hematopoietic stem cell" or "hematopoietic stem and progenitor cell."

As used herein, the terms "migration" and "mobilization" have the same meaning.

As used herein, the term "combination therapy" is used in the same sense as "combination." The term "combination therapy" means that two or more drugs are used together. As such, two or more drugs are not necessarily mixed as a composition and may be used as individual preparations. As such, "combination therapy" comprises individual preparations of two or more drugs such that simultaneous, separate or sequential administration of two or more drugs is possible.

The term "combination product" used in the present invention refers to the form of a finished product of the "combination therapy." For example, when there are two or more drugs, a combination product means a finished product comprising these drugs in the form of a combination. The two or more drugs may be packaged as a single preparation in a finished product and administered simultaneously or may be packaged in two individual preparations in a finished product and administered simultaneously, separately or sequentially. An example of such a finished product is a "kit-of-parts."

The term "subject" used in the present invention is an animal applicable to the method for collecting a large amount of mobilized hematopoietic stem cells of the present invention, preferably a vertebrate animal, more preferably a mammal, for example, animals comprising human beings, monkeys, dogs, cats, goats, pigs, rats, guinea pigs, hamsters, chimpanzees or gorillas. Most preferably, the subject is a human being.

In one embodiment of the present invention, in a combination therapy for mobilization of a large amount of hematopoietic stem cells in blood, comprising substance P and AMD3100 or a pharmaceutically acceptable salt thereof, substance P and AMD3100 may be sequentially administered. Specifically, substance P is first administered, and then AMD3100 is administered. More specifically, substance P is administered once or twice on two consecutive days, and AMD3100 is administered once about 1 to 2 days later.

In one embodiment of the present invention, in a combination product for mobilizing a large amount of hematopoietic stem cells in blood, comprising substance P and AMD3100 or a pharmaceutically acceptable salt thereof, substance P and AMD3100 may be sequentially administered. Specifically, substance P is first administered, and then AMD3100 is administered. More specifically, substance P is administered once or twice on two consecutive days, and AMD3100 is administered once about 1 to 2 days later.

In one embodiment of the present invention, the administration form of substance P and AMD3100 is not particularly limited and may be administered parenterally, preferably by injection, etc. Specifically, substance P is administered intravenously, and AMD3100 is administered intraperitoneally. In addition, substance P and AMD3100 may be administered once or several times in an amount sufficient to mobilize hematopoietic stem cells to peripheral blood in consideration of the type and condition of the subject to be administered.

In one embodiment of the present invention, substance P may be administered once or twice on two consecutive days at a dose of 0.1 to 100 µg/kg, preferably 1 to 20 µg/kg, and AMD3100 may be administered once or several times at a dose of 100 to 500 µg/kg, preferably 250 µg/kg. However, the form of administration, frequency of administration and dosage may be appropriately changed according to the judgment of those skilled in the art.

In one embodiment of the present invention, a combination product for mobilizing a large amount of hematopoietic stem cells in blood comprising substance P and AMD3100 or a pharmaceutically acceptable salt thereof may be in the form of a kit-of-parts.

In one embodiment of the present invention, in the method for collecting a large amount of mobilized hematopoietic stem cells, comprising separating blood fractions containing mobilized hematopoietic stem cells from peripheral blood obtained from a subject to which substance P and AMD3100 or a pharmaceutically acceptable salt thereof were sequentially administered, the step of separating the blood fractions may be performed using apheresis.

Substance P is a neuropeptide comprising eleven (11) amino acids expressed in sensory neurons, macrophages, eosinophils, endothelial cells, corneal cells such as epithelial cells and keratocytes, and granulation tissue. The substance P used in the present invention may be obtained through various routes, e.g., by synthesis through known amino acid sequence information, separation from a subject for use, and acquisition from a commercially available preparation. For example, commercially available substance P includes substance P acetate salt hydrate (sigma), substance p (Anygen), substance p (Merck Millipore), etc.

AMD3100, also called Plerixafor, is a compound whose compound name is 1,1'-[1,4-phenylenebis(methylene)]bis[1,4,8,11-tetraazacyclotetradecane] and is known to perform as an antagonist of CXCR4, a chemokine receptor *(see* US Patent No. 5,612,478).

AMD3100 used in the present invention may be obtained through various routes, e.g., by chemical synthesis methods known in the art and acquisition from commercially available preparations. For example, commercially available AMD3100 comprises Mozobil^{™} (Sanofi-Aventis), etc.

In addition, AMD3100 used in the present invention may be used in the form of a pharmaceutically acceptable salt. For example, a suitable acid addition salt comprises biocompatible inorganic acids (e.g., HCl, HBr, sulfuric acid and phosphoric acid), organic acids (e.g., acetic acid, propionic acid and butyric acid) and acids containing one or more carboxyl groups (e.g., oxalic acid, glutaric acid and adipic acid).

In addition, AMD3100 used in the present invention may be prepared and used in the form of a prodrug-that is, in a protected form that releases the compound of the present invention after administration to a subject. Also, when prepared in a purified form, the compound may crystallize as a hydrate.

A method for collecting a large amount of mobilized hematopoietic stem cells using the combination therapy comprising substance P and AMD3100 or a pharmaceutically acceptable salt thereof of the present invention specifically comprises (a) separating blood fractions containing mobilized hematopoietic stem cells from peripheral blood obtained from a subject to which substance P and AMD3100 or a pharmaceutically acceptable salt thereof were sequentially administered; and (b) concentrating the blood fractions.

In one embodiment of the present invention, the subject may be a vertebrate, more preferably a mammal comprising a human being. The hematopoietic stem cells collected in large quantities in the present invention are used to prevent or treat vascular diseases and, thus, are most preferably obtained from a patient to be treated.

In one embodiment of the present invention, in the above collection method, substance P is first administered, and AMD3100 is then administered, and blood is collected about 1 to 5 hours after the administration of AMD3100. More specifically, after substance P is administered once or twice on two consecutive days, AMD3100 is administered once about 1 to 2 days later, and blood is collected about 1 to 3 hours after the administration of AMD3100.

Separating the blood fractions from peripheral blood in step (a) may be performed using a method for collecting a blood component conventionally known in the art-for example, collecting the blood component by passing peripheral blood through an apheresis machine. Examples of the apheresis machine widely used in the art may be MCS 3p, CS-3000 plus, COBE Spectra, etc., but the present invention is not limited thereto. When blood enters the apheresis machine through the central venous catheter, blood fractions are collected by the machine, and the remaining blood may be returned to the body through the catheter. This process may take about 4 to 10 hours and may be repeated continuously for 2 to 10 days to collect cell fractions sufficient to be used for therapeutic purposes.

In one embodiment of the present invention, step (b) is a step of concentrating the blood fractions separated in step (a). Preferably, concentration of the blood fraction may be performed using a blood centrifuge. The conditions for centrifugation may be appropriately adjusted by those skilled in the art according to circumstances, and plasma components may be removed to obtain a concentrate (blood cell layer fractions) comprising monocyte components excluding erythrocytes. In a specific embodiment of the present invention, the blood fractions were placed on a Ficoll layer and centrifuged (813g, Megafuge 8R, Thermo Scientific) for 20 minutes to obtain a total of four layers, the uppermost layer being plasma, the second layer being a blood cell layer excluding erythrocytes, the third layer being Ficoll, and the bottom layer being an erythrocyte layer. Among these, the blood cell layer was obtained as a concentrate comprising a large amount of mobilized hematopoietic stem cells, but the present invention is not limited thereto.

Hereinafter, the constitution and effects of the present invention will be described in more detail by using working examples. These examples are merely examples to aid understanding of the present invention, and the scope of the present invention is not limited thereto.

### Example 1. Mobilization of hematopoietic stem cells by SP alone

In order to confirm the effect of the drug administered in a normal physiological state alone, the effect of SP was tested using a normal group without wounds. This is because, in the case of injured animals, there are many other factors secreted by the wounds, and, thus, it is difficult to differentiate such factors from the effect of the administered drug, and especially because both SP and G-CSF are endogenous substances in the body.

To confirm the effect of SP alone on mobilization of hematopoietic stem cell, five (5) rabbits were prepared for each group. SP (6.7 µg/kg, sigma Aldrich) was administered twice via Ear vein, and the levels of hematopoietic stem cells in the blood of the same animals for 3 days were compared. G-CSF (10 µg/kg, JW Pham) was used at a conventional clinical dose but treated with the same regimen as that of SP (i.e., intravenous administration).

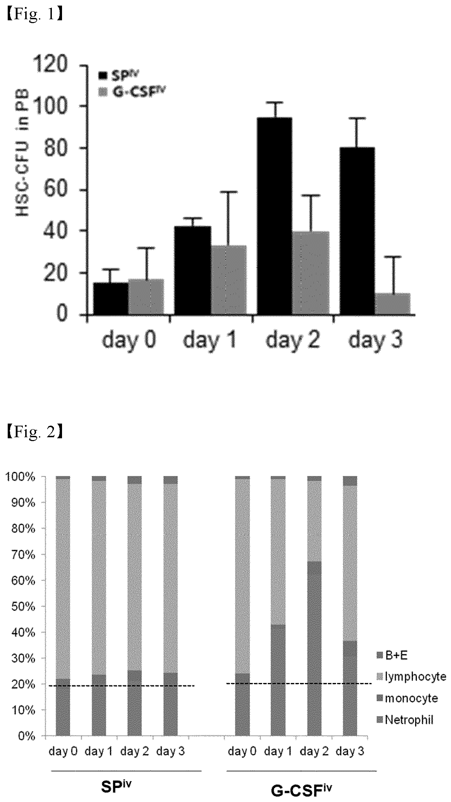

After injection of the drug, 5 ml of blood was collected once a day, put into a 15 ml tube (Falcon), and diluted twice by adding PBS (Welgene). To isolate blood cells, each of 5 ml Ficoll-Paque (Amersham) was placed in a new tube, and diluted blood was placed on top of it. The tubes were carefully handled so as not to mix the Ficoll layer and blood layer, and, after the blood layers were completely put in the tubes, centrifugation (813g, Megafuge 8R, Thermo Scientific) was performed for 20 minutes. After centrifugation, the layers were separated, resulting in a total of four layers. The uppermost layer was plasma, the second layer was the blood cell layer excluding erythrocytes, the third layer was Ficoll, and the bottom layer was the erythrocyte layer. Among them, only the blood cell layer was separated and put into a tube, and PBS was then added thereto to centrifuge same. 5×10⁶ blood cells were placed in a 35 mm plate and cultured in HSC-CFU media (Hematopoietic stem cells-colony forming unit media, purchased from Miltenyi). While the cells were cultured in the initial state for 2 weeks without exchanging the media, the number of colonies was observed every day and finally counted. The results are shown in FIG. 1.

As shown in FIG. 1, after injection of SP, the highest level of mobilization of hematopoietic stem cells was shown on the second day, which was maintained until the third day. On the other hand, G-CSF administered with the same regimen (i.e., intravenous administration) showed a negligible effect on mobilization of hematopoietic stem cells. Specifically, it seemed to increase on day 1 and did not increase further, while maintaining a similar level to that on day 0 (just before administration). Specifically, it was shown that SP was 3X (3 times), GCSF was 2X (2 times) on day 1; SP was 7X (7-fold), and GCSF was 2.5X (2.5-fold) on day 2; SP was 6X (6 times), and GCSF was 0.6X (0.6 times) on day 3, on the basis of 1X on day 0.

The main mechanism of G-CSF is the stimulation of neutrophil production. When the degree of mobilization of hematopoietic stem cells into blood by G-CSF at a concentration effective for mobilizing neutrophils to peripheral blood was compared with that by SP, it was found that SP induced mobilization of hematopoietic stem cells in blood more efficiently than G-CSF.

### Example 2. Effect of SP on blood immune cells

To confirm the effect of SP on the percentage of immune cells in blood, five (5) rabbits were prepared for each group. After administering SP (6.7 µg/kg) via ear vein twice, the percentages of immune cells in the blood of the same animals were compared for 3 days. G-CSF (10 µg/kg, JW Pham) was used at a conventional clinical dose and treated with the same regimen as that of SP (i.e., intravenous administration). After the administration, 1 ml of the blood was collected at one-day intervals, transferred to a tube containing EDTA and analyzed by using a diagnostic device.

The percentages of lymphocytes, monocytes, neutrophils, basophils and eosinophils in total leukocytes in the blood were analyzed by CBC count, and the results are shown in FIG. 2.

As shown in FIG. 2, although the administration of SP did not significantly affect the distribution of immune cells in the blood, G-CSF increased the percentage of neutrophils, the representative inflammatory cells in the blood, from day 1 after the administration and by about 65% on day 2.

These are the results of the normal group without wounds, which indicate that patients already have high levels of inflammation and, thus, may suffer from greater side effects when using G-CSF.

### Example 3. Mobilization of hematopoietic stem cells by SP and AMD3100 combination therapy

In order to compare the effects of G-CSF and SP on the basis of a clinical dose and regimen, five (5) mice of 8-week-old C57Bl/6 were prepared per group. The groups were divided into three groups: a group for administering a vehicle (saline), a group for administering G-CSF+AMD3100, and a group for administering SP+AMD3100.

G-CSF (250 µg/kg, subcutaneous, STEM CELL) and SP (6.7 µg/kg, intravenous, Sigma Aldrich) were administered to mice, respectively. G-CSF was administered subcutaneously for 5 consecutive days according to a conventional clinical schedule, and SP was administered intravenously for 2 consecutive days. AMD3100 was administered according to a conventional clinical schedule on the last day of each administration or one day later, and blood was collected 1 hour after the administration of AMD3100.

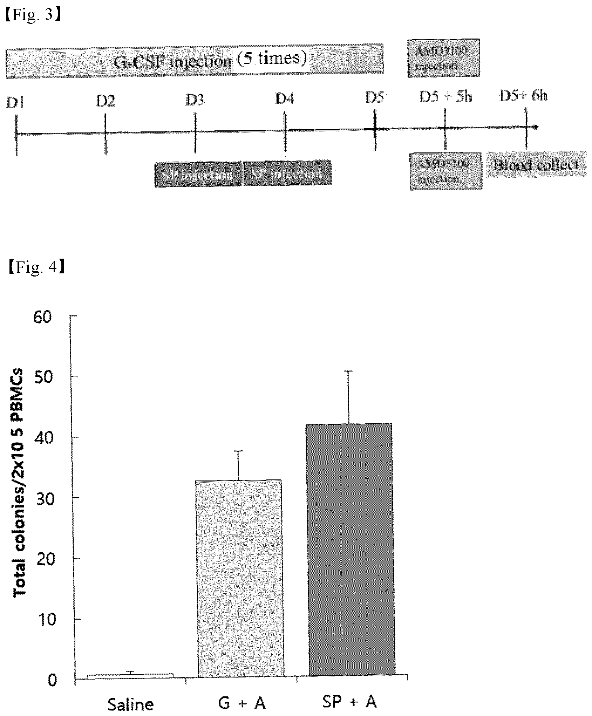

FIG. 3 is a schematic diagram showing the administration schedule of a conventional G-CSF and ADM3100 combination therapy and the administration schedule of substance P and AMD3100 combination therapy of the present invention.

After administering AMD3100, 1 ml of the mice's whole blood was separated using a syringe (containing heparin). The blood was placed in a 15 ml tube and diluted twice by adding PBS (Welgene). To isolate blood cells, each of 3 ml Ficoll-Paque (Amersham) was placed in a new tube, and diluted blood was placed on top of it. The tubes were carefully handled so as not to mix the Ficoll layer and blood layer, and, after the blood layers were completely put in the tubes, centrifugation (813g, Megafuge 8R, Thermo Scientific) was performed for 20 minutes. After centrifugation, the layers were separated, resulting in a total of four layers. The uppermost layer was plasma, the second layer was the blood cell layer excluding erythrocytes, the third layer was Ficoll, and the bottom layer was the erythrocyte layer. Among them, only the blood cell layer was separated and put into a tube, and PBS was then added thereto to centrifuge same. 2×10⁶ blood cells were placed in a 35 mm plate and cultured in HSC-CFU media (Hematopoietic stem cells-colony forming unit media, purchased from Miltenyi). While the cells were cultured in the initial state for 2 weeks without exchanging the media, the number of colonies was observed every day and finally counted. The results are shown in FIG. 4.

As shown in FIG. 4, the combination of G-CSF and AMD3100 promoted the migration of many hematopoietic stem cells into blood, as demonstrated clinically. However, the combination of SP and AMD3100 showed the migration of more hematopoietic stem cells. In particular, since G-CSF was administered 5 times, and SP was administered 2 times in the experiments of the present invention, the combination of SP and AMD3100 is regarded as showing a greater effect of mobilizing hematopoietic stem cells in blood according to the dose, as compared to the combination of G-CSF and AMD3100.

## Claims

1. A combined therapy for mobilization of a large amount of hematopoietic stem cells in blood, comprising substance-P and AMD 3100 or a pharmaceutically acceptable salt thereof.

2. The combined therapy according to Claim 1, wherein substance P and AMD3100 are sequentially administered.

3. The combined therapy according to Claim 1, wherein AMD3100 is administered once about 1 to 2 days later, after substance P is administered once.

4. The combined therapy according to Claim 1, wherein AMD3100 is administered once about 1 to 2 days later, after substance P is administered twice on two consecutive days.

5. The combined therapy according to any one of Claims 1-4, wherein substance P is administered by intravenous injection.

6. A combined product for mobilization of a large amount of hematopoietic stem cells in blood, comprising substance P and AMD 3100 or a pharmaceutically acceptable salt thereof.

7. The combined product according to Claim 6, wherein substance P and AMD3100 are sequentially administered.

8. The combined product according to Claim 6, wherein AMD3100 is administered once about 1 to 2 days later, after substance P is administered once or twice on two consecutive days.

9. The combined product according to Claim 6, wherein AMD3100 is administered once about 1 to 2 days later, after substance P is administered twice on two consecutive days.

10. The combined product according to any one of Claims 6-9, wherein substance P is administered by intravenous injection.

11. The combined product according to any one of Claims 6-9, wherein the combined product is in the form of a kit-of-parts.

12. A method for collecting a large amount of mobilized hematopoietic stem cells, comprising the step of separating blood fractions containing mobilized hematopoietic stem cells from peripheral blood obtained from a subject to which substance P and AMD3100 or a pharmaceutically acceptable salt thereof were sequentially administered.

13. The method according to Claim 12, wherein substance P is first administered, and AMD3 100 is then administered, and blood is collected about 1 to 5 hours after the administration of AMD3100.

14. The method according to Claim 12, wherein the step of separating the blood fractions is performed using apheresis.
